# EUROPÄISCHE PATENTANMELDUNG

(11) **EP 4 074 362 A1**
(43) Veröffentlichungstag der Anmeldung: **19.10.2022**
(21) Anmeldenummer: 22167554.9
(22) Anmeldetag: 11.04.2022
(51) Int. Cl.: A61M 25/02, A61M 25/00

(54) **HAUTPROTEKTOR ZUM SCHUTZ EINES KATHETERISIERTEN PATIENTEN**

(30) Priorität: 13.04.2021 LU 102778
(71) Anmelder: Koch, Silvia, 65719 Hofheim (DE)
(72) Erfinder: Koch, Silvia, 65719 Hofheim (DE)
(74) Vertreter: Hoffmann, Jürgen

(57) **Zusammenfassung**

Die Erfindung betrifft die Verwendung eines Hautprotektor, zum Bilden eines Kathetersystems, das einen Katheter, der den Katherterschlauch (3) aufweist, sowie den Hautprotektor aufweist, derart, dass der Katherterschlauch (3) beweglich in dem von der schlauchförmigen Hülle (2) umgebenen Raum angeordnet ist. Der Hautprotektor weist eine flexible, schlauchförmige Hülle zur Aufnahme eines Katheterschlauchs auf. Die flexible, schlauchförmige Hülle weist eine sich entlang ihrer Längserstreckungsrichtung erstreckende Öffnung auf, die mittels einer Verschlussvorrichtung verschließbar oder verschlossen ist.

## Beschreibung

Die Erfindung betrifft die Verwendung eines Hautprotektors.

Katheter, insbesondere Blasenkatheter, Blasenverweilkatheter und Drainagen, weisen zumeist einen flexiblen Schlauch auf, der durch eine natürliche oder künstliche Körperöffnung ins Körperinnere geführt ist. Ein Teil des Schlauchs verläuft außerhalb des Körpers zumeist zu einem Gefäß, das dazu ausgebildet ist, Flüssigkeiten aufzunehmen. Oftmals werden Katheter mehrere Tage oder sogar mehrere Wochen verwendet. Insbesondere bei Patienten, die nicht in der Lage sind, die Toilette aufzusuchen, werden Blasenkatheter, insbesondere Blasenverweilkatheter, zur Ableitung des Urins aus der Harnblase oftmals über lange Zeiträume oder dauerhaft eingesetzt.

Es hat sich gezeigt, dass es oftmals zu Hauterkrankungen in den Körperbereichen kommt, in denen der außerhalb des Körpers verlaufende Teil des Katheterschlauchs verläuft. Die Hauterkrankungen reichen von Hautirritationen bis hin zu Decubitus und Hautblutungen.

Es ist bekannt, Katheterschläuche, insbesondere Drainageschläuche, mit einem einfachen Pflaster oder durch spezielle Vorrichtungen an der Haut des Patienten punktuell zu fixieren. Allerdings dienen diese Fixierungen lediglich dazu, ein Verheddern des Katheterschlauchs zu verhindern. Die oben genannten Hauterkrankungen werden hierdurch nicht verhindert. Im Gegenteil verursachen die zum Fixieren verwendeten Pflaster und die zumeist auf Klebebasis funktionierenden Vorrichtungen selbst ungewollte Hautreaktionen.

Beispielsweise ist aus DE 201 21 558 U1 eine Vorrichtung zur Fixierung eines Drainageschlauchs einer Wunddrainage bekannt. Die Vorrichtung weist ein Halterelement auf, das als Ringschelle ausgebildet ist und Befestigungsflügel mit einer Selbstklebeschicht aufweist.

Aus DE 10 2006 020 406 A1 ist eine Drainageschlauchfixiervorrichtung mit einem Basiselement und einem am Basiselement angeordneten Halterelement für wenigstens einen Drainageschlauch bekannt. Das Basiselement weist auf seiner zur Festlegung an der Haut eines Patienten bestimmten Seite eine Klebstoffschicht auf.

Es ist daher die Aufgabe der vorliegenden Erfindung, eine Möglichkeit anzugeben, eine Hautschädigung bei einem katheterisierten Patienten zu vermeiden.

Die Aufgabe wird durch die Verwendung eines Hautprotektors gelöst, der eine flexible, schlauchförmige Hülle zur Aufnahme eines Katheterschlauchs aufweist, die eine sich entlang ihrer Längserstreckungsrichtung erstreckende Öffnung aufweist, und der wenigstens eine Verschlussvorrichtung aufweist, mittels der die Öffnung zumindest teilweise verschließbar oder verschlossen ist, , zum Bilden eines Kathetersystems, das einen Katheter, der den Katherterschlauch (3) aufweist, sowie den Hautprotektor aufweist, derart, dass der Katherterschlauch (3) beweglich in dem von der schlauchförmigen Hülle (2) umgebenen Raum angeordnet ist.

In erfindungsgemäßer Weise wurde erkannt, dass die Hautschädigungen bei katheterisierten Patienten insbesondere auf sich ständig wiederholende Relativbewegungen des Katheterschlauchs relativ zur Haut des Patienten zurückzuführen sind. Hierbei kommt erschwerend hinzu, dass das Material (oft Silikon oder ähnliches) des Katheterschlauchs relativ zur Haut zumeist keinen geringen Reibungswiderstand aufweist.

Der erfindungsgemäß verwendete Hautprotektor hat den ganz besonderen Vorteil, dass der außerhalb des Körpers verlaufende Teil eines Katheterschlauchs in der schlauchförmigen Hülle des Hautprotektors verläuft und daher ein direkter Kontakt mit der Haut des Patienten wirkungsvoll vermieden ist. Vielmehr kann sich der außerhalb des Körpers verlaufende Teil eines Katheterschlauchs innerhalb des Hautprotektors bewegen, während der Hautprotektor ruhig an der Haut des Patienten anliegen kann. Darüber hinaus kann der erfindungsgemäße Hautprotektor problemlos aus einem hautsympathischen Material, beispielsweise aus einem Mikrofaserstoff, gefertigt werden.

Es hat sich bei Versuchen gezeigt, dass die Hautschädigungen bei bereits geschädigten Patienten während des Einsatzes des Hautprotektors innerhalb kurzer Zeit vollständig verschwanden. Insbesondere war Linderung der geschädigten Haut zumeist schon nach einigen Stunden sichtbar. Bei neu katheterisierten Patienten, die noch keine Hautschädigungen aufwiesen, konnten durch den Einsatz des Hautprotektors auch über längere Zeiträume Hautschädigungen vollständig vermieden werden. Insoweit eignet sich der erfindungsgemäße Hautprotektor ganz besonders als Präventivmaßnahme zur Vermeidung von Hautschädigungen.

Insbesondere kann der erfindungsgemäß verwendete Hautprotektor einfach zum Einsatz gebracht werden, indem der außerhalb des Körpers verlaufende Teil eines Katheterschlauchs einfach, schnell und effizient über die Öffnung in den Hautprotektor eingelegt und die schlauchförmige Hülle mittels der Verschlussvorrichtung anschließend verschlossen wird. Insbesondere ist es vorteilhaft möglich, den Hautprotektor bei einem bereits katheterisierten Patienten anzulegen, ohne hierfür den Katheter von dem Patienten entfernen oder manipulieren zu müssen. Darüber hinaus ist es vorteilhaft möglich, den Hautprotektor (beispielsweise für einen Waschvorgang oder um ihn durch einen frischen Hautprotektor zu ersetzen) wieder zu entfernen, ohne hierfür den Katheter entfernen oder manipulieren zu müssen.

Vorzugsweise erstreckt sich die mittels der wenigstens eine Verschlussvorrichtung verschließbare oder verschlossene Öffnung von einem Ende bis zum anderen Ende der Hülle. Dies ermöglicht es, die schlauchförmige Hülle vollständig ihrer gesamten Länge nach öffnen zu können, um einen Katheterschlauch einlegen zu können oder um einen Katheterschlauch wieder herausnehmen zu können.

Bei einer ganz besonders vorteilhaften Ausführung weist die schlauchförmige Hülle wenigstens eine Revisionsöffnung auf. Eine solche Ausführung ist ganz besonders vorteilhaft, weil sie es dem Patienten, dem Pfleger und/oder dem Arzt ermöglicht, den (zumeist durchsichtigen) Katheterschlauch und/oder dessen Inhalt zu inspizieren. Beispielsweise ist es bei einem Blasenkatheter wichtig fortlaufend zu prüfen, ob es Urinveränderungen gibt. Insbesondere kann vorteilhaft vorgesehen sein, dass die schlauchförmige Hülle mehrere Revisionsöffnungen entlang ihrer Längserstreckungsrichtung aufweist. Auf diese Weise ist sichergestellt, dass sämtliche Abschnitte des innerhalb des Hautprotektors verlaufenden Katheterschlauchs inspiziert werden können.

Insbesondere kann die Revisionsöffnung vorteilhaft als Schlitz ausgebildet sein. Eine solche Ausführung hat den ganz besonderen Vorteil, dass die Schlitzränder für einen Inspektionsvorgang einfach vorübergehend auseinandergezogen werden können, während sie ansonsten automatisch dicht aneinander oder übereinander liegen und ein Kontakt des Katheterschlauchs mit der Haut des Patienten durch die Revisionsöffnung hindurch vermieden ist. Insbesondere hierfür ist es von besonderem Vorteil, wenn sich die Revisionsöffnung entlang der Längserstreckungsrichtung der schlauchförmigen Hülle erstreckt.

Die Revisionsöffnung kann insbesondere als eine von der Öffnung separate weitere Öffnung ausgebildet sein. Es ist vorteilhaft allerdings auch möglich, dass die Revisionsöffnung durch einen nicht verschließbaren Teil der Öffnung der schlauchförmigen Hülle gebildet ist. Beispielsweise kann die Verschlussvorrichtung mehrere Verschlüsse aufweisen, mittels denen die Öffnung verschließbar oder verschlossen ist, wobei die Verschlüsse entlang der Öffnung voneinander beabstandet angeordnet sind, so dass zwischen zwei unmittelbar benachbarten Verschlüssen im verschlossenen Zustand der schlauchförmigen Hülle jeweils eine Revisionsöffnung ausgebildet ist. Eine solche Ausführung ist ganz besonders vorteilhaft, weil die Verschlussvorrichtung mit sehr geringem Materialaufwand herstellbar ist und gleichzeitig, ohne dass ein zusätzlicher Arbeitsschritt erforderlich ist, automatisch wenigstens eine Revisionsöffnung vorhanden ist (vorzugsweise mehrere Revisionsöffnungen vorhanden sind).

Natürlich ist es auch möglich, dass die Revisionsöffnung durch einen nicht verschließbaren Teil der Öffnung der schlauchförmigen Hülle gebildet und dass zusätzlich wenigstens eine weitere, von der Öffnung der schlauchförmigen Hülle separate Revisionsöffnung vorhanden ist.

Bei einer ganz besonders vorteilhaften Ausführung ist die Verschlussvorrichtung, insbesondere nicht zerstörungsfrei lösbar, an der schlauchförmigen Hülle befestigt. Eine solche Ausführung hat beispielsweise den besonderen Vorteil, dass der Hautprotektor einteilig ausgebildet sein kann, ohne dass zum Verschließen zusätzliche und nicht mit der schlauchförmigen Hülle verbundene Bauteile verwendet werden müssen. Beispielsweise kann die Verschlussvorrichtung ein Klettband und ein Flauschband aufweisen, die beide an die Hülle angenäht und oder angeklebt und oder angenietet sind.

Hinsichtlich der Art der Verschlussvorrichtung gibt es keine grundsätzlichen Beschränkungen.

Ganz allgemein kann die Verschlussvorrichtung wenigstens einen Klettverschluss aufweisen. Insbesondere kann die Verschlussvorrichtung mehrere Klettverschlüsse aufweisen. Bei einer Ausführung, bei der die mehreren Klettverschlüsse entlang der Längserstreckungsrichtung der Hülle voneinander beabstandet sind, sind (wie oben bereits beschrieben) in vorteilhafter Weise zwischen den Klettverschlüssen automatisch Revisionsöffnungen realisiert.

Alternativ kann die Verschlussvorrichtung als Klebeverschlussvorrichtung ausgebildet sein. Insbesondere kann die Klebeverschlussvorrichtung einen Kleber aufweisen, der ein zerstörungsfreies lösen ermöglicht. Alternativ kann auch vorgesehen sein, dass die Klebeverschlussvorrichtung einen Kleber aufweist, der kein zerstörungsfreies Öffnen der schlauchförmigen Hülle erlaubt. Eine solche Ausführung bietet sich an, wenn der Hautprotektor als Einmalprodukt ausgebildet ist und sichergestellt werden soll, dass nur eine einmalige Verwendung stattfindet.

Die Verschlussvorrichtung kann beispielsweise wenigstens einen Knopf und wenigstens ein Knopfloch aufweisen. Insbesondere kann die Verschlussvorrichtung auch wenigstens einen Druckknopf aufweisen. Alternativ oder zusätzlich kann die Verschlussvorrichtung eine Schnürung aufweisen.

Bei einer ganz besonders vorteilhaften Ausführung ist die Verschlussvorrichtung im verschlossenen Zustand der schlauchförmigen Hülle vollständig im Inneren der schlauchförmigen Hülle angeordnet. Alternativ oder zusätzlich kann vorteilhaft vorgesehen sein, dass die Verschlussvorrichtung im verschlossenen Zustand der schlauchförmigen Hülle nicht nach außen über die Außenseite der schlauchförmigen Hülle hinaus ragt und/der dass die Verschlussvorrichtung im verschlossenen Zustand der schlauchförmigen Hülle verdeckt ist. Diese Ausführungen haben den ganz besonderen Vorteil, dass ein Kontakt der Haut des Patienten mit der Verschlussvorrichtung vermieden ist. Auf diese Weise ist sichergestellt, dass die Verschlussvorrichtung keine Hautschädigungen bei dem Patienten hervorruft.

Die schlauchförmige Hülle kann vorteilhaft wenigstens teilweise aus einem Textilmaterial hergestellt sein. Bei einer ganz besonders vorteilhaften und insbesondere hautfreundlichen Ausführung ist die schlauchförmige Hülle wenigstens teilweise aus einem Mikrofaserstoff hergestellt. Die Verwendung eines Mikrofaserstoffs hat darüber hinaus den ganz besonderen Vorteil, dass eine besonders leichte Ausführung des Hautprotektors ermöglicht ist. Darüber hinaus hat ein Mikrofaserstoff den besonderen Vorteil, dass er nicht zum Ausfransen neigt, so dass auf eine Behandlung der Ränder, beispielsweise durch Ketteln, verzichtet werden kann.

Die schlauchförmige Höhe kann insbesondere aus einem wenigstens teilweise luftdurchlässigen Material, insbesondere Textilmaterial, hergestellt sein. Auf diese Weise ist es ermöglicht, dass die Haut des Patienten "atmen" kann.

Alternativ oder zusätzlich kann die schlauchförmige Hülle aus einem waschbaren, insbesondere maschinenwaschbaren, Material, insbesondere Textilmaterial, hergestellt sein. Dies ermöglicht es, den Hautprotektor nach einem Waschvorgang wieder verwenden zu können.

Von besonderem Vorteil ist es, wenn die schlauchförmige Hülle aus einem nicht ausfransenden Material, insbesondere Textilmaterial, hergestellt ist. Der Vorteil liegt insbesondere darin, dass auf eine Behandlung der Ränder, beispielsweise durch Ketteln, verzichtet werden kann. Darüber hinaus ist vorteilhaft vermieden, dass es zu einer Hautschädigung durch die behandelten Ränder kommt.

Bei einer ganz besonders vorteilhaften Ausführung ist die schlauchförmige Hülle wenigstens teilweise aus einem länglichen, insbesondere rechteckigen, Streifen eines flexiblen Materials, insbesondere eines Textilmaterials, gebildet. Hierbei kann vorteilhaft insbesondere vorgesehen sein, dass die beiden sich entlang der Längserstreckungsrichtung erstreckenden Randbereiche des länglichen Streifens mittels der Verschlussvorrichtung miteinander verbindbar sind, um die schlauchförmige Hülle zu schließen. Hierzu kann die Verschlussvorrichtung, insbesondere nicht zerstörungsfrei lösbar, an den beiden sich entlang der Längserstreckungsrichtung erstreckenden Randbereichen des länglichen Streifens befestigt sein.

Der Streifen kann eine erste Seite, die im verschlossenen Zustand des Hautprotektors wenigstens einen Teil der Innenseite der schlauchförmigen Hülle bildet, sowie eine zweite Seite, die im verschlossenen Zustand des Hautprotektors wenigstens einen Teil der Außenseite der schlauchförmigen Hülle bildet, aufweisen. Bei einer ganz besonders vorteilhaften Ausführung ist die Verschlussvorrichtung auf der zweiten Seite an den beiden sich entlang der Längserstreckungsrichtung erstreckenden Randbereiche befestigt. Zusätzlich kann vorteilhaft vorgesehen sein, dass die beiden sich entlang der Längserstreckungsrichtung erstreckenden Randbereiche derart umgeschlagen sind, dass sich die Umschlagkanten, insbesondere parallel zueinander, entlang der Längserstreckungsrichtung erstrecken. Auf diese Weise ist vorteilhaft erreicht, dass die schlauchförmige Hülle verschlossen werden kann, ohne dass Teile der Verschlussvorrichtung nach außen ragen. Dies hat den besonderen Vorteil, dass die Verschlussvorrichtung nicht in Kontakt mit der Haut des Patienten gelangen kann.

Der Hautprotektor kann, je nach Anwendung, eine Länge im Bereich von 20 cm bis 100 cm, insbesondere im Bereich von 50 cm bis 90 cm, insbesondere von 70 cm, aufweist.

Der Hautprotektor weist vorteilhaft im verschlossenen und flachen Zustand eine Breite im Bereich von 3 cm bis 10 cm, insbesondere im Bereich von 5cm bis 9 cm, insbesondere von 7 cm, auf. Eine solche Breite ermöglicht eine begrenzte Bewegungsfähigkeit des Katheterschlauchs innerhalb des Hautprotektors. Auf diese Weise sind Verspannungen des Katheterschlauchs vermieden. Außerdem hat eine solche Breite den besonderen Vorteil, dass ein effizientes Inspizieren durch die, insbesondere schlitzförmigen Revisionsöffnungen, problemlos möglich ist. Die genannte Breite hat darüber hinaus den ganz besonderen Vorteil, dass nicht nur der Katheterschlauch, sondern andere Teile des Katheters, die an dem Katheterschlauch angeordnet sind oder mit diesem verbunden sind, beispielsweise Verzweigungsstücke, Kupplungsstücke oder Anschlussstücke, in dem Hautprotektor angeordnet werden können.

Insbesondere für eine Anwendung in Krankenhäusern und Pflegeanstalten und für die häusliche Pflege ist ein Hautprotektorspendermaterial von ganz besonderem Vorteil, von dem, je nach Bedarf in der erforderlichen Länge jeweils ein erfindungsgemäßer und vorzugsweise unmittelbar gebrauchsfertiger Hautprotektor abgetrennt, insbesondere abgeschnitten, werden kann.

Von ganz besonderem Vorteil ist ein Spender, der mit Hautprotektorspendermaterial ausgerüstet ist. Beispielsweise kann der Spender eine Wickelrolle aufweisen, auf die das Hautprotektorspendermaterial aufgewickelt ist. Der Arzt oder der Pfleger kann jeweils einen Teil des Hautprotektorspendermaterials von der Wickelrolle abwickeln und dann einen Hautprotektor in der erforderlichen Länge von dem restlichen Hautprotektorspendermaterial, vorzugsweise durch einfaches Abschneiden mit einer Schere, abtrennen.

Alternativ oder zusätzlich kann der Spender auch ein Gehäuse aufweisen, in dem das Hautprotektorspendermaterial angeordnet, insbesondere aufgerollt, ist, wobei ein Ende des Hautprotektorspendermaterials zum Herausziehen durch eine Gehäuseöffnung des Gehäuses ragt. Der Arzt oder der Pfleger kann jeweils einen Teil des Hautprotektorspendermaterials durch die Gehäuseöffnung aus dem Gehäuse herausziehen und dann einen Hautprotektor in der erforderlichen Länge von dem restlichen Hautprotektorspendermaterial, vorzugsweise durch einfaches Abschneiden mit einer Schere, abtrennen.

Von ganz besonderem Vorteil ist ein Kathetersystem, das einen Katheter, der einen Katherterschlauch aufweist, sowie einen erfindungsgemäßen Hautprotektor aufweist.

Hinsichtlich der Art der Katheter, mit denen der erfindungsgemäße Hautprotektor verwendet werden kann, gibt es keine grundsätzlichen Beschränkungen. Insbesondere kann es sich um einen Blasenkatheter handeln. Es ist auch möglich, dass es sich um eine Drainage, beispielsweise eine Saugdrainage, wie beispielsweise eine Redon-Drainage, handelt.

Es ist ein Hautprotektor bzw. eine Hautprotektorspendermaterial bzw. ein Spender und ein Kathetersystem, insbesondere für die erfindungsgemäße Verwendung, besonders vorteilhaft, der bzw. das wenigstens einen der nachfolgend genannten Aspekte aufweist:
1. Hautprotektor (1) zum Schutz eines katheterisierten Patienten, aufweisend eine flexible, schlauchförmige Hülle (2) zur Aufnahme eines Katheterschlauchs (3), die eine sich entlang ihrer Längserstreckungsrichtung erstreckende Öffnung (4) aufweist, und aufweisend wenigstens eine Verschlussvorrichtung, mittels der die Öffnung (4) zumindest teilweise verschließbar oder verschlossen ist.
2. Hautprotektor (1) nach Aspekt 1, dadurch gekennzeichnet, dass sich die mittels der wenigstens einen Verschlussvorrichtung verschließbare oder verschlossene Öffnung (4) von einem Ende bis zum anderen Ende der Hülle (2) erstreckt.
3. Hautprotektor (1) nach Aspekt 1 oder 2, dadurch gekennzeichnet, dass die schlauchförmige Hülle (2) wenigstens eine Revisionsöffnung (5) aufweist.
4. Hautprotektor (1) nach Aspekt 3, dadurch gekennzeichnet, dass durch die Revisionsöffnung (5) hindurch ein in der Hülle (2) angeordneter Katheterschlauch (3) und/oder dessen Inhalt inspizierbar ist.
5. Hautprotektor (1) nach Aspekt 3 oder 4, dadurch gekennzeichnet, dass die Revisionsöffnung (5) als Schlitz ausgebildet ist.
6. Hautprotektor (1) nach einem der Aspekte 3 bis 5, dadurch gekennzeichnet, dass sich die Revisionsöffnung (5) entlang der Längserstreckungsrichtung der schlauchförmige Hülle (2) erstreckt.
7. Hautprotektor (1) nach einem der Aspekte 3 bis 6, dadurch gekennzeichnet, dass die Revisionsöffnung (5) als eine von der Öffnung (4) separate weitere Öffnung ausgebildet ist.
8. Hautprotektor (1) nach einem der Aspekte 1 bis 7, dadurch gekennzeichnet, dass die Verschlussvorrichtung mehrere Verschlüsse aufweist, mittels denen die Öffnung verschließbar oder verschlossen ist.
9. Hautprotektor (1) nach Aspekt 8 sowie einem der Aspekte 3 bis 6, dadurch gekennzeichnet, dass sich die Verschlüsse entlang der Öffnung voneinander beabstandet angeordnet sind und dass zwischen zwei unmittelbar benachbarten Verschlüssen im verschlossenen Zustand der schlauchförmigen Hülle (2) jeweils eine Revisionsöffnung (5) ausgebildet ist.
10. Hautprotektor (1) nach einem der Aspekte 1 bis 9, dadurch gekennzeichnet, dass die Verschlussvorrichtung, insbesondere nicht zerstörungsfrei lösbar, an der schlauchförmigen Hülle (2) befestigt ist.
11. Hautprotektor (1) nach einem der Aspekte 1 bis 10, dadurch gekennzeichnet, dass die Verschlussvorrichtung wenigstens einen Klettverschluss (6) aufweist.
12. Hautprotektor (1) nach Aspekt 11, dadurch gekennzeichnet, dass die Verschlussvorrichtung ein Klettband (9) und ein Flauschband (8) aufweist.
13. Hautprotektor (1) nach einem der Aspekte 1 bis 10, dadurch gekennzeichnet, dass die Verschlussvorrichtung als, insbesondere zerstörungsfrei wieder lösbare, Klebeverschlussvorrichtung ausgebildet ist.
14. Hautprotektor (1) nach einem der Aspekte 1 bis 10, dadurch gekennzeichnet, dass die Verschlussvorrichtung einen Knopf und/oder einen Druckknopf aufweist.
15. Hautprotektor (1) nach einem der Aspekte 1 bis 10, dadurch gekennzeichnet, dass die Verschlussvorrichtung eine Schnürung aufweist.
16. Hautprotektor (1) nach einem der Aspekte 1 bis 15, dadurch gekennzeichnet, dass die Verschlussvorrichtung im verschlossenen Zustand der schlauchförmigen Hülle (2) vollständig im Inneren der schlauchförmigen Hülle (2) angeordnet ist.
17. Hautprotektor (1) nach einem der Aspekte 1 bis 16, dadurch gekennzeichnet, dass die Verschlussvorrichtung im verschlossenen Zustand der schlauchförmigen Hülle (2) nicht nach außen über die Außenseite der schlauchförmigen Hülle (2) hinaus ragt.
18. Hautprotektor (1) nach einem der Aspekte 1 bis 17, dadurch gekennzeichnet, dass die Verschlussvorrichtung im verschlossenen Zustand der schlauchförmigen Hülle (2) verdeckt ist.
19. Hautprotektor (1) nach einem der Aspekte 1 bis 18, dadurch gekennzeichnet, dass die schlauchförmige Hülle (2) wenigstens teilweise aus einem Textilmaterial hergestellt ist.
20. Hautprotektor (1) nach einem der Aspekte 1 bis 19, dadurch gekennzeichnet, dass die schlauchförmige Hülle (2) wenigstens teilweise aus einem Mikrofaserstoff hergestellt ist.
21. Hautprotektor (1) nach einem der Aspekte 1 bis 20, dadurch gekennzeichnet, dass die schlauchförmige Hülle (2) aus einem wenigstens teilweise luftdurchlässigen Material, insbesondere Textilmaterial, hergestellt ist.
22. Hautprotektor (1) nach einem der Aspekte 1 bis 21, dadurch gekennzeichnet, dass die schlauchförmige Hülle (2) aus einem waschbaren, insbesondere maschinenwaschbaren, Material, insbesondere Textilmaterial, hergestellt ist.
23. Hautprotektor (1) nach einem der Aspekte 1 bis 22, dadurch gekennzeichnet, dass die schlauchförmige Hülle (2) aus einem nicht ausfransendem Material, insbesondere Textilmaterial, hergestellt ist.
24. Hautprotektor (1) nach einem der Aspekte 1 bis 23, dadurch gekennzeichnet, dass die schlauchförmige Hülle (2) wenigstens teilweise aus einem länglichen Streifen (17) eines flexiblen Materials, insbesondere eines Textilmaterials, gebildet ist.
25. Hautprotektor (1) nach Aspekt 24, dadurch gekennzeichnet, dass die beiden sich entlang der Längserstreckungsrichtung erstreckenden Randbereiche (7) des länglichen Streifens (17) mittels der Verschlussvorrichtung miteinander verbindbar sind, um die schlauchförmige Hülle (2) zu schließen.
26. Hautprotektor (1) nach Aspekt 25, dadurch gekennzeichnet, dass die Verschlussvorrichtung, insbesondere nicht zerstörungsfrei lösbar, an den beiden sich entlang der Längserstreckungsrichtung erstreckenden Randbereichen (7) des länglichen Streifens (17) befestigt ist.
27. Hautprotektor (1) nach einem der Aspekte 24 bis 26, dadurch gekennzeichnet, dass der Streifen (17) eine erste Seite (10), die im verschlossenen Zustand des Hautprotektors (1) wenigstens einen Teil der Innenseite der schlauchförmigen Hülle (2) bildet, sowie eine zweite Seite (11), die im verschlossenen Zustand des Hautprotektors (1) wenigstens einen Teil der Außenseite der schlauchförmigen Hülle (2) bildet, aufweist.
28. Hautprotektor (1) nach Aspekt 27, dadurch gekennzeichnet, dass die Verschlussvorrichtung auf der zweiten Seite (11) an den beiden sich entlang der Längserstreckungsrichtung erstreckenden Randbereichen (7) befestigt ist.
29. Hautprotektor (1) nach Aspekt 28, dadurch gekennzeichnet, dass die beiden sich entlang der Längserstreckungsrichtung erstreckenden Randbereiche (7) derart umgeschlagen sind, dass sich die Umschlagkanten (12), insbesondere parallel zueinander, entlang der Längserstreckungsrichtung erstrecken.
30. Hautprotektor (1) nach einem der Aspekte 1 bis 29, dadurch gekennzeichnet, dass der Hautprotektor (1) eine Länge im Bereich von 20 cm bis 100 cm, insbesondere im Bereich von 50 cm bis 90 cm, insbesondere von 70 cm, aufweist.
31. Hautprotektor (1) nach einem der Aspekte 1 bis 30, dadurch gekennzeichnet, dass der Hautprotektor (1) verschlossenen, flachen Zustand eine Breite im Bereich von 3 cm bis 10 cm, insbesondere im Bereich von 5cm bis 9 cm, insbesondere von 7 cm, aufweist.
32. Hautprotektor (1) nach einem der Aspekte 1 bis 31, dadurch gekennzeichnet, dass der Hautprotektor (1) einteilig ausgebildet ist.
33. Hautprotektorspendermaterial (15), von dem nacheinander mehrere unmittelbar gebrauchsfertige Hautprotektoren (1) nach einem der Aspekte 1 bis 32 abtrennbar, insbesondere abschneidbar, sind.
34. Spender (14), der mit Hautprotektorspendermaterial (15) nach Aspekt 33 ausgerüstet ist.
35. Spender (14) nach Aspekt 34, gekennzeichnet durch eine Wickelrolle (16), auf die das Hautprotektorspendermaterial (15) aufgewickelt ist.
36. Spender (14) nach Aspekt 35, gekennzeichnet durch ein Gehäuse, in dem das Hautprotektorspendermaterial (15) angeordnet, insbesondere aufgerollt, ist, wobei ein Ende des Hautprotektorspendermaterials (15) zum Herausziehen durch eine Gehäuseöffnung des Gehäuses ragt.
37. Kathetersystem umfassend einen Katheter, der einen Katherterschlauch (3) aufweist, sowie einen Hautprotektor (1) nach einem der Aspekte 1 bis 32.

In der Zeichnung ist der Erfindungsgegenstand beispielhaft und schematisch dargestellt und wird anhand der Figuren nachfolgend beschrieben, wobei gleiche oder gleich wirkende Elemente auch in unterschiedlichen Ausführungsbeispielen zumeist mit denselben Bezugszeichen versehen sind. Dabei zeigen:
- Fig. 1: schematisch ein erstes Ausführungsbeispiel eines Hautprotektors für eine erfindungsgemäße Verwendung im verschlossenen Zustand,
- Fig. 2: schematisch ein zweites Ausführungsbeispiel eines Hautprotektors für eine erfindungsgemäße Verwendung im verschlossenen Zustand,
- Fig. 3: das zweite Ausführungsbeispiel bei der Inspektion des Katheterschlauchs,
- Fig. 4: das zweite Ausführungsbeispiel im geöffnetem Zustand,
- Fig. 5: schematisch eine Illustration einer erfindungsgemäßen Verwednung eines Hautprotektors, und
- Fig. 6: eine Arztpraxis mit einem Spender für Hautprotektorspendermaterial, von dem ein erfindungsgemäßer Hautprotektor für eine erfindungsgemäße Verwendung abtrennbar ist.

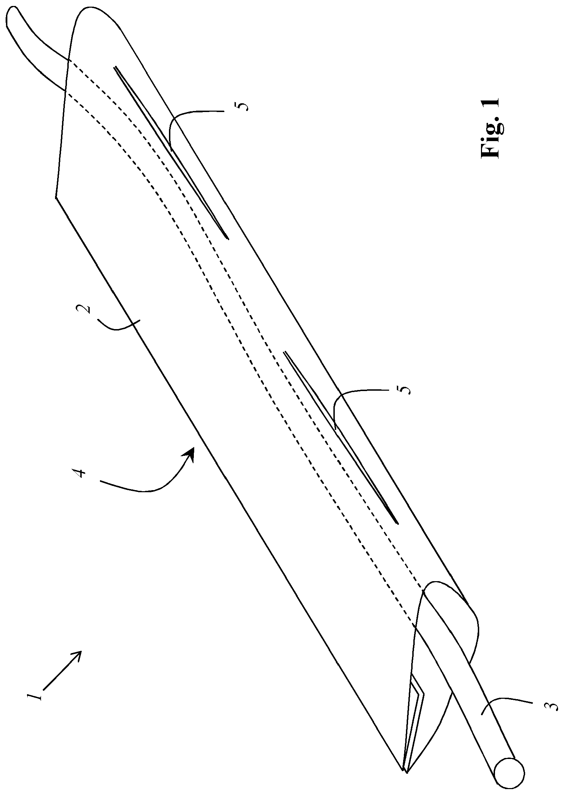

Fig. 1 zeigt einen Hautprotektor 1 zum Schutz eines katheterisierten Patienten. Der Hautprotektor 1 weist eine flexible, schlauchförmige Hülle 2 zur Aufnahme eines Katheterschlauchs 3 auf. Die flexible, schlauchförmige Hülle 2 weist eine sich entlang ihrer Längserstreckungsrichtung erstreckende Öffnung 4 auf, die mittels einer (in Figur 1 nicht dargestellten) Verschlussvorrichtung verschlossen ist.

Die schlauchförmige Hülle 2 weist entlang ihrer Längserstreckungsrichtung mehrere schlitzförmige Revisionsöffnungen 5 auf, die es dem Patienten, dem Pfleger und/oder dem Arzt ermöglichen, den Katheterschlauch 3 und/oder dessen Inhalt zu inspizieren. Bei der Inspektion kann der der Hautprotektor 1 relativ zu dem Katheterschlauch 3 entlang der Längserstreckungsrichtung einfach etwas verschoben werden, um sämtliche Abschnitte des Katheterschlauchs 3 in inspizieren zu können.

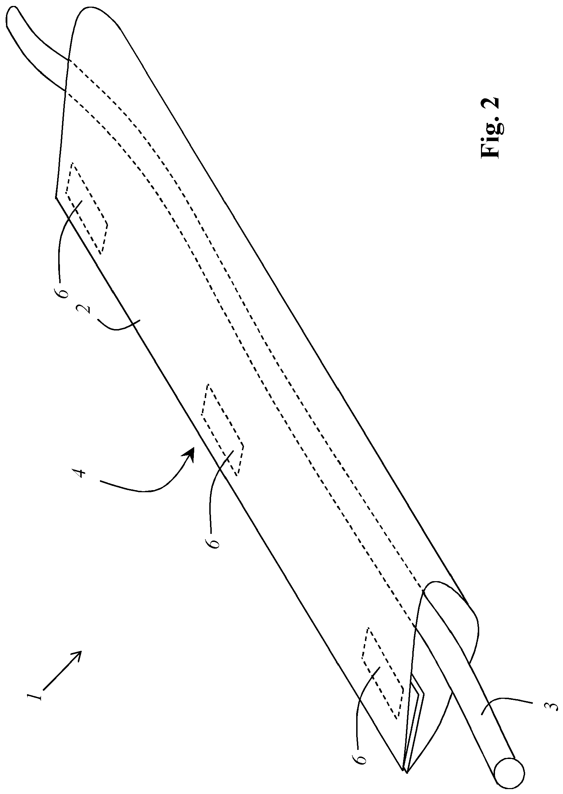

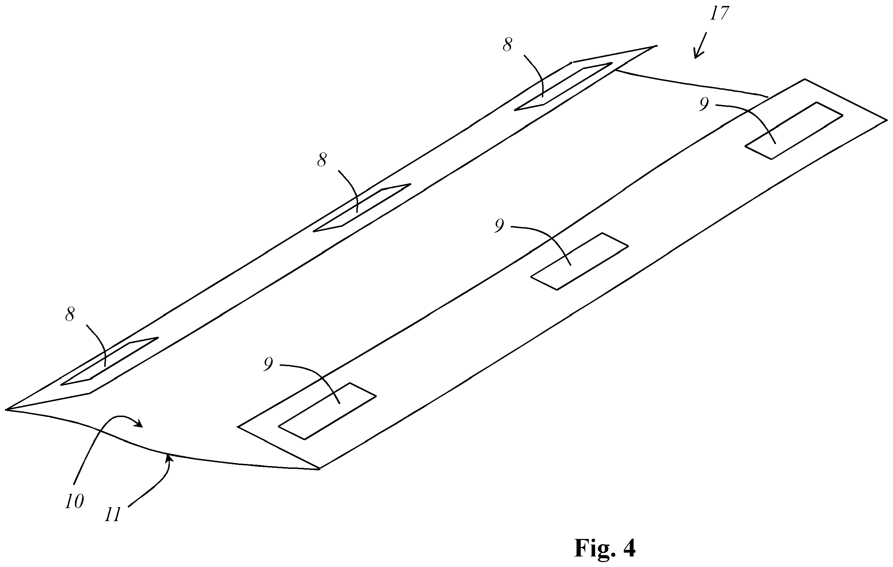

Die Figuren 2 bis 4 zeigen schematisch ein zweites Ausführungsbeispiel eines erfindungsgemäßen Hautprotektors 1. Die Figur 2 zeigt das zweite Ausführungsbeispiel eines erfindungsgemäßen Hautprotektors 1 im verschlossenen Zustand.

Die flexible, schlauchförmige Hülle 2 weist eine sich entlang ihrer Längserstreckungsrichtung erstreckende Öffnung 4 auf, die mittels einer Verschlussvorrichtung verschlossen ist, die mehrere voneinander beabstandete Klettverschlüsse 6 aufweist. Die Klettverschlüsse 6 sind entlang der Öffnung 4 voneinander beabstandet angeordnet, so dass zwischen zwei unmittelbar benachbarten Klettverschlüssen 6 jeweils eine Revisionsöffnung 5 ausgebildet ist.

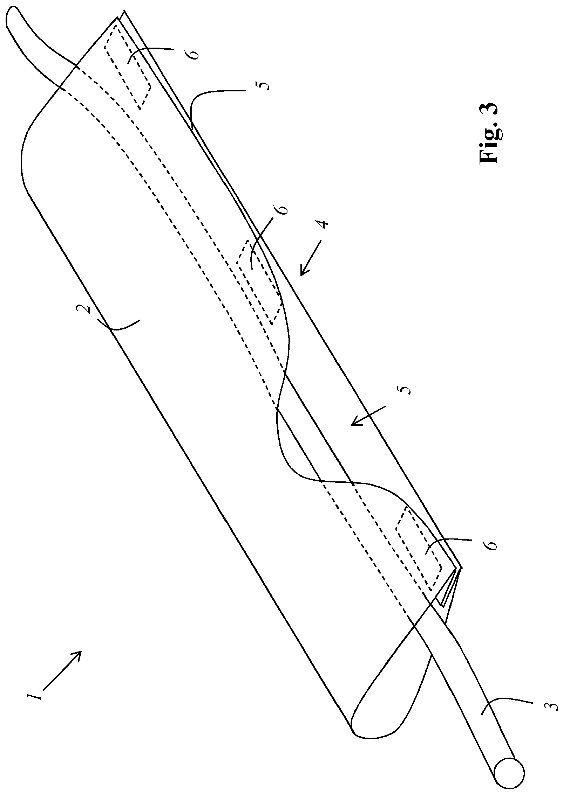

Figur 3 zeigt das zweite Ausführungsbeispiel (von der anderen Seite aus gesehen) bei der Inspektion des Katheterschlauchs 3. in Figur 3 ist besonders gut zu erkennen, wie die Revisionsöffnungen 5 ausgebildet und angeordnet sind.

Figur 3 zeigt das zweite Ausführungsbeispiel im geöffneten Zustand. Die schlauchförmige Hülle 2 weist einen länglichen, rechteckigen Streifen 17 eines flexiblen Materials, insbesondere eines Textilmaterials, auf, wobei die beiden sich entlang der Längserstreckungsrichtung erstreckenden Randbereiche 7 des länglichen Streifens 17 mittels der Verschlussvorrichtung 6 miteinander verbindbar sind, um die schlauchförmige Hülle 2 zu schließen. Hierzu sind an einem der Randbereiche 7 Flauschbänder 8 und an dem anderen der Randbereiche 7 Klettbänder 9 angenäht.

Der Streifen weist eine erste Seite 10, die im verschlossenen Zustand des Hautprotektors 1 wenigstens einen Teil der Innenseite der schlauchförmigen Hülle 2 bildet, sowie eine zweite Seite 11, die im verschlossenen Zustand des Hautprotektors 1 wenigstens einen Teil der Außenseite der schlauchförmigen Hülle 2 bildet, auf.

Die Flauschbänder 8 und die Klettbänder 9 sind auf der zweiten Seite 11 an die beiden sich entlang der Längserstreckungsrichtung erstreckenden Randbereiche 7 angenähert. Die beiden sich entlang der Längserstreckungsrichtung erstreckenden Randbereiche 7 sind derart umgeschlagen, dass sich die Umschlagkanten 12 entlang der Längserstreckungsrichtung erstrecken. Auf diese Weise ist vorteilhaft erreicht, dass die schlauchförmige Hülle 2 durch Verbinden jeweils eines der Flauschbänder 8 mit einem der Klettbänder 9 verschlossen werden kann, ohne dass im Ergebnis Teile der Flauschbänder 8 oder der Klettbänder 9 nach außen ragen. Dies hat den besonderen Vorteil, dass die Verschlussvorrichtung nicht in Kontakt mit der Haut des Patienten gelangen kann.

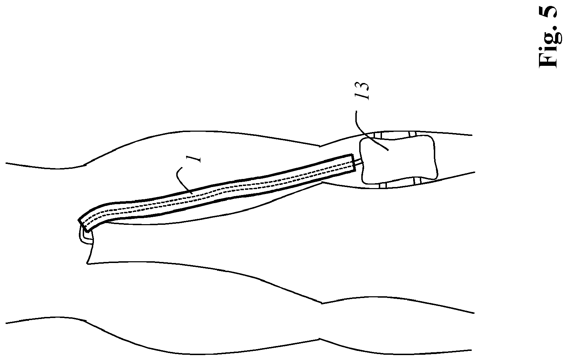

Figur 5 zeigt schematisch eine Illustration der Anwendung eines erfindungsgemäßen Hautprotektors 1 bei einem Patienten, der mit einem Blasenkatheter katheterisiert ist. Der Katheterschlauch 3 endet an einem Beutel 13, der am Unterschenkel des Patienten befestigt ist. Nahezu der gesamte außerhalb des Körpers liegende Abschnitt des Katheterschlauchs 3 ist innerhalb des Hautprotektors 1 angeordnet und kann daher nicht mit der Haut des Patienten in Kontakt kommen. Auf diese Weise sind Hautschäden wirkungsvoll vermieden.

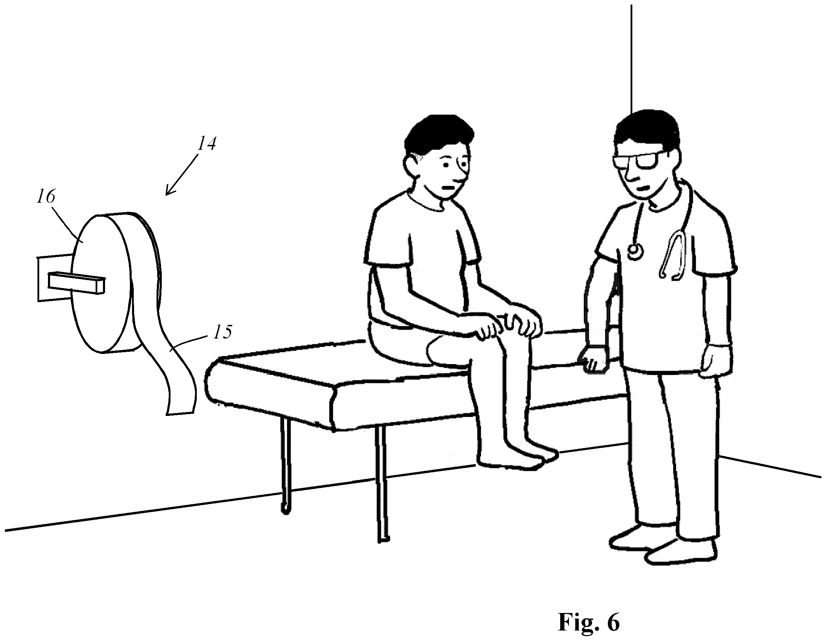

Figur 6 zeigt exemplarisch eine Arztpraxis mit einem Spender 14 für Hautprotektorspendermaterial 15, von dem ein erfindungsgemäßer Hautprotektor abtrennbar ist. Der Spender weist eine an der Wand gehaltert Wickelrolle 16 auf, auf die das Hautprotektorspendermaterial 15 aufgewickelt ist. Der Arzt oder der Pfleger kann jeweils einen Teil des Hautprotektorspendermaterials 15 von der Wickelrolle 16 abwickeln und dann einen Hautprotektor 1 in der erforderlichen Länge von dem restlichen Hautprotektorspendermaterial 15, vorzugsweise durch einfaches Abschneiden mit einer Schere, abtrennen. Anschließend kann der abgetrennte Hautprotektor 1 bei der Versorgung des Patienten verwendet werden.

### Bezugszeichenliste:

- 1: Hautprotektor
- 2: schlauchförmige Hülle
- 3: Katheterschlauch
- 4: Öffnung
- 5: Revisionsöffnung
- 6: Klettverschluss
- 7: Randbereich
- 8: Flauschband
- 9: Klettband
- 10: erste Seite
- 11: zweite Seite
- 12: Umschlagkanten
- 13: Beutel
- 14: Spender
- 15: Hautprotektorspendermaterial
- 16: Wickelrolle
- 17: Streifen

## Patentansprüche

1. Verwendung eines Hautprotektors (1), der eine flexible, schlauchförmige Hülle (2) zur Aufnahme eines Katheterschlauchs (3) aufweist, die eine sich entlang ihrer Längserstreckungsrichtung erstreckende Öffnung (4) aufweist, und der wenigstens eine Verschlussvorrichtung aufweist, mittels der die Öffnung (4) zumindest teilweise verschließbar oder verschlossen ist, zum Bilden eines Kathetersystems, das einen Katheter, der den Katherterschlauch (3) aufweist, sowie den Hautprotektor aufweist, derart, dass der Katherterschlauch (3) beweglich in dem von der schlauchförmigen Hülle (2) umgebenen Raum angeordnet ist.

2. Verwendung nach Anspruch 1, **dadurch gekennzeichnet, dass** sich die mittels der wenigstens einen Verschlussvorrichtung verschließbare oder verschlossene Öffnung (4) von einem Ende bis zum anderen Ende der Hülle (2) erstreckt.

3. Verwendung nach Anspruch 1 oder 2, **dadurch gekennzeichnet, dass**
a. die schlauchförmige Hülle (2) wenigstens eine Revisionsöffnung (5) aufweist, und/oder dass
b. die schlauchförmige Hülle (2) wenigstens eine Revisionsöffnung (5) aufweist und durch die Revisionsöffnung (5) hindurch ein in der Hülle (2) angeordneter Katheterschlauch (3) und/oder dessen Inhalt inspizierbar ist, und/oder dass
c. die schlauchförmige Hülle (2) wenigstens eine Revisionsöffnung (5) aufweist, die als Schlitz ausgebildet ist, und/oder dass
d. die schlauchförmige Hülle (2) wenigstens eine Revisionsöffnung (5) aufweist, die sich entlang der Längserstreckungsrichtung der schlauchförmige Hülle (2) erstreckt. Und/oder dass
e. die schlauchförmige Hülle (2) wenigstens eine Revisionsöffnung (5) aufweist, die als eine von der Öffnung (4) separate weitere Öffnung ausgebildet ist.

4. Verwendung nach einem der Ansprüche 1 bis 3, **dadurch gekennzeichnet, dass**
a. die Verschlussvorrichtung mehrere Verschlüsse aufweist, mittels denen die Öffnung verschließbar oder verschlossen ist, oder dass
b. die Verschlussvorrichtung mehrere Verschlüsse aufweist, mittels denen die Öffnung verschließbar oder verschlossen ist, wobei sich die Verschlüsse entlang der Öffnung voneinander beabstandet angeordnet sind und dass zwischen zwei unmittelbar benachbarten Verschlüssen im verschlossenen Zustand der schlauchförmigen Hülle (2) jeweils eine Revisionsöffnung (5) ausgebildet ist.

5. Verwendung nach einem der Ansprüche 1 bis 4, **dadurch gekennzeichnet, dass**
a. die Verschlussvorrichtung, insbesondere nicht zerstörungsfrei lösbar, an der schlauchförmigen Hülle (2) befestigt ist, und/oder dass
b. die Verschlussvorrichtung wenigstens einen Klettverschluss (6) aufweist, und/oder dass
c. die Verschlussvorrichtung als, insbesondere zerstörungsfrei wieder lösbare, Klebeverschlussvorrichtung ausgebildet ist, und/oder dass
d. die Verschlussvorrichtung einen Knopf und/oder einen Druckknopf aufweist, und/oder dass
e. die Verschlussvorrichtung eine Schnürung aufweist.

6. Verwendung nach einem der Ansprüche 1 bis 5, **dadurch gekennzeichnet, dass**
a. die Verschlussvorrichtung im verschlossenen Zustand der schlauchförmigen Hülle (2) vollständig im Inneren der schlauchförmigen Hülle (2) angeordnet ist, und/oder dass
b. die Verschlussvorrichtung im verschlossenen Zustand der schlauchförmigen Hülle (2) nicht nach außen über die Außenseite der schlauchförmigen Hülle (2) hinaus ragt, und/oder dass
c. die Verschlussvorrichtung im verschlossenen Zustand der schlauchförmigen Hülle (2) verdeckt ist.

7. Verwendung nach einem der Ansprüche 1 bis 6, **dadurch gekennzeichnet, dass**
a. die schlauchförmige Hülle (2) wenigstens teilweise aus einem Textilmaterial hergestellt ist, und/oder dass
b. die schlauchförmige Hülle (2) wenigstens teilweise aus einem Mikrofaserstoff hergestellt ist, und/oder dass
c. die schlauchförmige Hülle (2) aus einem wenigstens teilweise luftdurchlässigen Material, insbesondere Textilmaterial, hergestellt ist, und/oder dass
d. die schlauchförmige Hülle (2) aus einem waschbaren, insbesondere maschinenwaschbaren, Material, insbesondere Textilmaterial, hergestellt ist, und/oder dass
e. die schlauchförmige Hülle (2) aus einem nicht ausfransendem Material, insbesondere Textilmaterial, hergestellt ist.

8. Verwendung nach einem der Ansprüche 1 bis 7, **dadurch gekennzeichnet, dass**
a. die schlauchförmige Hülle (2) wenigstens teilweise aus einem länglichen Streifen (17) eines flexiblen Materials, insbesondere eines Textilmaterials, gebildet ist,oder dass
b. die schlauchförmige Hülle (2) wenigstens teilweise aus einem länglichen Streifen (17) eines flexiblen Materials, insbesondere eines Textilmaterials, gebildet ist und die beiden sich entlang der Längserstreckungsrichtung erstreckenden Randbereiche (7) des länglichen Streifens (17) mittels der Verschlussvorrichtung miteinander verbindbar sind, um die schlauchförmige Hülle (2) zu schließen oder dass
c. die schlauchförmige Hülle (2) wenigstens teilweise aus einem länglichen Streifen (17) eines flexiblen Materials, insbesondere eines Textilmaterials, gebildet ist und die beiden sich entlang der Längserstreckungsrichtung erstreckenden Randbereiche (7) des länglichen Streifens (17) mittels der Verschlussvorrichtung miteinander verbindbar sind, um die schlauchförmige Hülle (2) zu schließen, wobei die Verschlussvorrichtung, insbesondere nicht zerstörungsfrei lösbar, an den beiden sich entlang der Längserstreckungsrichtung erstreckenden Randbereichen (7) des länglichen Streifens (17) befestigt ist.

9. Verwendung nach Anspruch 8, **dadurch gekennzeichnet, dass** der Streifen (17) eine erste Seite (10), die im verschlossenen Zustand des Hautprotektors (1) wenigstens einen Teil der Innenseite der schlauchförmigen Hülle (2) bildet, sowie eine zweite Seite (11), die im verschlossenen Zustand des Hautprotektors (1) wenigstens einen Teil der Außenseite der schlauchförmigen Hülle (2) bildet, aufweist und die Verschlussvorrichtung auf der zweiten Seite (11) an den beiden sich entlang der Längserstreckungsrichtung erstreckenden Randbereichen (7) befestigt ist, wobei die beiden sich entlang der Längserstreckungsrichtung erstreckenden Randbereiche (7) derart umgeschlagen sind, dass sich die Umschlagkanten (12), insbesondere parallel zueinander, entlang der Längserstreckungsrichtung erstrecken.

10. Verwendung nach einem der Ansprüche 1 bis 9, **dadurch gekennzeichnet, dass**
a. der Hautprotektor (1) eine Länge im Bereich von 20 cm bis 100 cm, insbesondere im Bereich von 50 cm bis 90 cm, insbesondere von 70 cm, aufweist, und/oder dass
b. Verwendung nach einem der Ansprüche 1 bis 30, **dadurch gekennzeichnet, dass** der Hautprotektor (1) verschlossenen, flachen Zustand eine Breite im Bereich von 3 cm bis 10 cm, insbesondere im Bereich von 5cm bis 9 cm, insbesondere von 7 cm, aufweist, und/oder dass
c. Verwendung nach einem der Ansprüche 1 bis 31, **dadurch gekennzeichnet, dass** der Hautprotektor (1) einteilig ausgebildet ist.

11. Hautprotektorspendermaterial (15), von dem nacheinander mehrere unmittelbar gebrauchsfertige Hautprotektoren (1) für eine Verwendung nach einem der Ansprüche 1 bis 10 abtrennbar, insbesondere abschneidbar, sind.

12. Spender (14), der mit Hautprotektorspendermaterial (15) nach Anspruch 11 ausgerüstet ist.

13. Spender (14) nach Anspruch 12, **gekennzeichnet durch**
a. eine Wickelrolle (16), auf die das Hautprotektorspendermaterial (15) aufgewickelt ist, und/oder durch
b. ein Gehäuse, in dem das Hautprotektorspendermaterial (15) angeordnet, insbesondere aufgerollt, ist, wobei ein Ende des Hautprotektorspendermaterials (15) zum Herausziehen durch eine Gehäuseöffnung des Gehäuses ragt.

14. Kathetersystem umfassend einen Katheter, der einen Katherterschlauch (3) aufweist, sowie einen Hautprotektor (1), der eine flexible, schlauchförmige Hülle (2) zur Aufnahme des Katheterschlauchs (3) aufweist, die eine sich entlang ihrer Längserstreckungsrichtung erstreckende Öffnung (4) aufweist, und der wenigstens eine Verschlussvorrichtung aufweist, mittels der die Öffnung (4) zumindest teilweise verschließbar oder verschlossen ist, wobei der Katherterschlauch (3) beweglich in dem von der schlauchförmigen Hülle (2) umgebenen Raum verläuft.

15. Kathetersystem nach Anspruch 14, **dadurch gekennzeichnet, dass** der Hautprotektor (1) die zusätzlichen Merkmale wenigstens eines der Ansprüche 2 bis 10 aufweist.
